# EUROPÄISCHE PATENTSCHRIFT

(11) **EP 4 523 643 B1**
(45) Veröffentlichungstag und Bekanntmachung des Hinweises auf die Patenterteilung: **26.08.2026**
(21) Anmeldenummer: 23198033.5
(22) Anmeldetag: 18.09.2023
(51) Int. Cl.: A61B 17/86, A61B 50/30

(54) **ELASTISCH AUFWEITBARE STERILISIERBARE VERPACKUNG FÜR EINZELGEGENSTÄNDE**
ELASTICALLY EXPANDABLE SINGLE-PIECE STERILIZABLE PACKAGE
EMBALLAGE STÉRILISABLE ÉLASTIQUEMENT EXTENSIBLE POUR ARTICLES INDIVIDUELS

(43) Veröffentlichungstag der Anmeldung: 19.03.2025
(73) Patentinhaber: Rösler IP GmbH, 88138 Hergensweiler (DE)
(72) Erfinder: Rösler, Thiemo, 88239 Wangen/Neuravensburg (DE)
(74) Vertreter: Riebling, Peter

(56) Entgegenhaltungen:
- DE-A1- 102013 019 452
- DE-U1- 202014 100 438
- US-A- 4 892 525
- US-A1- 2017 095 308

## Beschreibung

Gegenstand der Erfindung ist eine elastisch aufweitbare sterilisierbare Verpackung für die Verpackung singulärer Gegenstände im medizinischen Bereich nach dem Oberbegriff des Patentanspruchs 1.

Eine solche elastisch aufweitbare Einzelverpackung dient als Verpackung für sterile medizinische Gegenstände, die insbesondere in der Chirurgie, der Orthopädie und weiteren medizinischen Bereichen verwendet werden. Solche Gegenstände müssen während der Operation - möglichst einhändig - einfach, störungsfrei und unter Beibehaltung der Sterilität aus der Verpackung entnehmbar sein.

Zweck der Einzelverpackung nach dem Stand der Technik und der vorliegenden Erfindung ist die Bereitstellung einer sterilisierbaren Verpackung für langgestreckte Gegenständen gleich welcher Art, die bevorzugt durch Strahlung sterilisierbar ist.

Aus der DE 10 2013 019 452 B4 ist eine elastisch aufweitbare Einzelverpackung für langgestreckte Gegenstände bekannt geworden, wobei der verpackte Gegenstand aus mindestens einem Kopf vergrößerten Durchmessers und einem sich mit vermindertem Durchmesser anschließendem Bolzenteil besteht. Die Einzelverpackung ist aus einer mindestens einseitig stirnseitig offenen, etwa zylindrischen Verpackungshülse gebildet, die aus einem elastisch biegbaren Material besteht, die mindestens eine obere Einführöffnung zum Einführen oder Entnehmen des langgestreckten Gegenstandes aufweist, wobei lediglich der Kopf des zu haltenden Gegenstandes im Bereich der Einführöffnung der Verpackung lagengesichert gehalten wird.

Als nachteilig wird angesehen, dass die bekannte Einzelverpackung auf die Halterung des Kopfes eines langgestreckten Gegenstandes - z.B. einer Schraube - angewiesen ist, was den Anwendungsbereich stark einschränkt. Andere Gegenstände, die einen solchen Kopf mit vergrößertem Durchmesser nicht aufweisen, können nicht lagengesichert gehalten werden.

Weiterer Nachteil der bekannten Einzelverpackung ist, dass die Einzelverpackung individuell an den Durchmesser des Schraubenkopfes angepasst werden muss, was dazu führt, dass eine Vielzahl von Einzelverpackungen hergestellt werden müssen, die an unterschiedliche Schraubenformen und Schraubenlängen sowie Kopfdurchmesser angepasst sind.

Es ist nur ein geringflächiger Griffbereich vorhanden, der nur im Bereich des aufweitbaren Halses der Einzelverpackung vorhanden ist, was dazu führt, dass die Griffflächen klein und dadurch schwer zu betätigen sind.

Nachdem der Schraubenkopf der zu haltenden Schraube im Bereich der Einführöffnung liegt, besteht die Gefahr, dass in der sterilen Umgebung des Operationssaals ein Bediener den Schraubenkopf unbeabsichtigt berührt und damit kontaminiert, was zu schweren Operationsschäden führen kann.

Bei der bekannten Einzelverpackung bestand demnach eine erhöhte Gefahr der Kontamination, weil der Schraubenkopf von der oberen Einführöffnung her zugänglich war, was zu vermeiden ist.

Bei der bekannten Einzelverpackung kann nur jeweils ein einziger langgestreckter Gegenstand verpackt werden, wobei vorausgesetzt ist, dass ein Schraubenkopf oder ein Kopf erweiterten Durchmessers vorhanden sind, weil die Haltefunktion in diesem Bereich stattfindet. Es handelte sich daher nur um einen etwa linienförmigen, elastisch aufweitbaren Klemmbund, der die Haltefunktion verwirklichte. Die Betätigung dieses Klemmbundes war - mangels ausreichender Griffflächen - ungünstig.

Im Übrigen ist die bekannte Einzelverpackung darauf angewiesen, dass eine bestimmte Kopfform vorgegeben ist, andernfalls die Verpackung nicht funktioniert.

Der Erfindung liegt deshalb die Aufgabe zugrunde, eine Verpackung für singuläre Gegenstände der eingangs genannten Art so weiterzubilden, dass die zu verpackenden Gegenstände betriebssicher in einer elastisch aufweitbaren Verpackung eingebracht werden können, und dass diese Gegenstände gegen unbeabsichtigte Berührung geschützt sind.

Zur Lösung der gestellten Aufgabe ist die Erfindung durch die technische Lehre des unabhängigen Patentanspruchs gekennzeichnet.

Dabei wird es bevorzugt, wenn im Übergangsbereich zwischen dem Verschlussteil und dem Verpackungsraum ein oder mehrere Sperrnoppen angeordnet sind, die zwischen sich einen elastisch aufweitbaren Verschlussspalt bilden und dass im Verpackungsraum eine zum Verschlussspalt in vertikaler Linie fluchtende Führungsvorrichtung zur lagengesicherten Führung des unteren Teils des verpackten Gegenstandes angeordnet ist.

Mit der Anordnung einer verpackungsraumseitigen Führungsvorrichtung zur verkantungsfreien Führung des unteren Teils des zu haltenden Gegenstandes wird der Vorteil erzielt, dass der untere Teil des Gegenstandes in einer Gleitführung mit radialem Spiel gehalten ist und sich nicht im Verpackungsraum verkanten kann.

Dabei wird es bevorzugt, wenn die Führungsvorrichtung aus zwei vertikalen, zueinander parallelen und einen gegenseitigen Abstand zueinander einnehmenden Begrenzungsrippen besteht, die zwischen sich einen etwa hülsenförmigen Führungskanal ausbilden, indem der untere Teil des zu haltenden Gegenstandes mit radialem Spiel in der Art einer Gleitführung lagengesichert gehalten ist.

Die Profilform des Führungskanals ist bevorzugt an die Form des zu haltenden Gegenstandes in seinem unteren Bereich angepasst. Bei einem rundprofilierten Gegenstand wird deshalb ein rundprofilierter Führungskanal vorgeschlagen. Ist jedoch der Gegenstand in seinem unteren Teil z. B. mehrkantig oder asymmetrisch ausgebildet, wird es bevorzugt, wenn auch der Führungskanal diesem mehrkantigen oder asymmetrischen Profil angepasst ist, sodass der zu haltenden Gegenstand mit Gleitspiel und verdrehungsgesichert im Führungskanal gehalten ist. Damit kann der Gegenstand nur in einer einzigen Verdrehlage aus der Einzelverpackung entnommen werden.

Um zu vermeiden, dass die Führungsvorrichtung elastisch verformt wird, wird es bevorzugt, wenn die zueinander parallelen vertikalen Begrenzungsrippen starr ausgebildet sind, was dadurch erreicht wird, dass die Begrenzungsrippen bevorzugt über ihre gesamte Länge die Vorderwand mit der Rückwand des Verpackungskörpers verbinden und so relativ starr und biegesteif ausgebildet sind.

Insgesamt handelt es sich um eine flache Verpackungstasche, in deren Verpackungsraum ein singulärer Gegenstand lagengesichert und verkantungsfrei gelagert ist und der durch Fingerdruck auf die Griffflächen über den sich dann öffnenden Verschlussspalt entnommen werden kann.

Die Entnahme kann dadurch erfolgen, dass die Einzelverpackung auf den Kopf gestellt wird und danach der Verschlussspalt durch Fingerdruck auf die Griffflächen geöffnet wird, wonach ein oder mehrere Gegenstände aufgrund der Schwerkraft herausfallen.

In einer anderen Variante kann der Gegenstand bei aufrechter Verpackung und geöffnetem Verschlussspalt mit einem Magnetwerkzeug oder einem geeigneten Greifwerkzeug aus dem Verpackungsraum entnommen werden.

Die Anordnung von Sperrnoppen, die den Verpackungsraum nach oben hin begrenzen und den Verschlussspalt ausbilden hat den Vorteil, dass bei einer Fehlbedienung, wenn beispielsweise die medizinische Hilfskraft die Verpackung Überkopf umgedreht hält und entleeren will, vermieden wird, dass die im Verpackungsraum gelagerten Gegenstände unbeabsichtigt herausfallen. Die erst durch Fingerdruck zu betätigenden Sperrnoppen, die den Verschlussspalt bilden, sind eine Sicherung gegen unbeabsichtigtes Entleeren.

Dabei wird es bevorzugt, wenn insgesamt ein elastisch aufweitbarer, flacher Verpackungskörper vorhanden ist, dessen oberes Verschlussteil elastisch aufweitbar ist, wobei das Verschlussteil durch mindestens zwei auf den Schmalseiten des Verpackungskörpers gegenüber liegend angeordnete Griffrippen gebildet ist, die gegeneinander elastisch gedrückt werden können, wodurch der im unteren Bereich des Verschlussteils angeordnete, durch ein oder mehrere Sperrnoppen gebildete Verschlussspalt aufweitet.

Durch den aufgeweiteten Verschlussspalt kann der im Verpackungsraum lagengesichert gehaltene Gegenstand betriebssicher aus der Verpackung entnommen werden, wenn diese mit der Entnahmeöffnung nach unten gerichtet ist. Alternativ kann er bei aufrechter Verpackung mit einem geeigneten Werkzeug entnommen werden.

Dabei wird es bevorzugt, wenn die ein oder mehreren Sperrnoppen jeweils innenliegend auf der Vorderwand und der Rückwand des Verschlussteils gegeneinander gerichtet und zueinander fluchtend angeordnet sind, wodurch sie sich bei geeignetem Verformungsdruck auf die einander gegenüberliegenden Griffflächen voneinander entfernen und dadurch einen geöffneten Verschlussspalt bilden, durch den hindurch der Gegenstand eingebracht oder entnommen werden kann.

Nach der Erfindung wird es demnach bevorzugt, wenn der obere Verschlussteil des Verpackungskörpers durch eine durch Fingerkraft aufweitbare Öffnung begrenzt ist, und dass jenseits von dieser Öffnung eine oder mehrere gegenüberliegende jeweils fluchtend auf der Vorder- und der Rückwand angeordnete Sperrnoppen angeordnet sind, die zwischen sich einen aufweitbaren Verschlussspalt bilden.

Mit der gegebenen technischen Lehre ergibt sich der Vorteil, dass der zu verpackende Gegenstand nicht mehr in notwendiger Weise eine Schraube mit einem im Durchmesser vergrößerten Schraubenkopf sein muss, sondern es können beliebig geformte Gegenstände sein, die durch die im oberen Bereich aufgeweitete Öffnung der Verpackung und durch den darunter liegenden aufgeweiteten Verschlussspalt hindurch in den unteren Verpackungsraum des Verpackungskörpers gelangen, ohne dass im oberen Bereich der Öffnung des Verpackungskörpers eine Berührungsgefahr für den Gegenstand besteht.

Damit können verbesserte sterile Anforderungen erfüllt werden und es kann ein singulärer Gegenstand sicher verpackt werden, weil er bei geschlossenem Verschlussspalt berührungssicher im Verpackungsbereich gehalten ist.

Hier unterscheidet sich die Erfindung vom Stand der Technik nach der DE10 2013 019 452 B4, denn die Form der Verpackung musste an die Form des zu haltenden Gegenstandes angepasst werden. Bei der Erfindung ist dies nicht mehr notwendig. Damit entfällt auch die Notwendigkeit, eine Vielzahl von Verpackungsvarianten für eine Vielzahl von unterschiedlichen, zu verpackenden singulären Gegenständen bereitzustellen, weil in einer einzigen Verpackung unterschiedliche singuläre Gegenständen verpackt werden können. Bei einem solchen Gegenstand kann es sich um einen Stift, eine Schraube, eine Mutter, eine Platte oder andere im medizinischen Bereich vorhandene kleinteiligen Gegenstände handeln.

Jeder singuläre Gegenstand kann bei aufgeweiteter Öffnung der Verpackung durch den im Abstand unter der Verpackungsmündung angeordneten, aufweitbaren Verschlussspalt hindurchpassen und kann bei geschlossenem Verschlussspalt nicht mehr die Verpackung verlassen.

Die Zuhaltekraft für die den Verschlussspalt bildenden Sperrnoppen wird durch die elastische Rückstellkraft des Kunststoffmaterials im Halsbereich der Verpackung definiert.

In einer anderen Ausgestaltung der Erfindung kann es noch zusätzlich vorgesehen sein, dass die Zuhaltekraft (Schliesskraft) des Verschlussspaltes dadurch vergrößert ist, dass die Sperrnoppen einen Verzahnungseingriff bilden.

Weil durch die technische Lehre die Erfindung es nun erstmals möglich ist, einen geschützten Verpackungsraum in der Einzelverpackung zu bilden, indem ein Gegenstand lagengesichert aufbewahrt werden kann, ergibt sich der weitere Vorteil, dass es im oberen Bereich, das heißt jenseits der Sperrnoppen und demnach im Bereich des oberen Randes erstmals möglich ist, die gesamte Öffnung durch Verschlussmittel sicher zu verschließen.

Ein erster sicherer Verschluss des Öffnungsrandes ist beispielsweise, dass das Kunststoffmaterial, aus dem der Verpackungskörper besteht, im Wege einer Verschweißung verschweißt werden kann, um so eine absolut dichte Verpackung zu schaffen, was bei der bekannten Einzelverpackung nach dem Stand der Technik nicht möglich war.

Eine weitere Verschlussmöglichkeit ist zum Beispiel, dass die Öffnung des Verpackungskörpers im oberen Bereich des umlaufenden Randes mit einem gesiegelten und abreißbaren Verschlussblatt oder mit einem Verschlusskleber verschlossen werden kann.

Wichtig bei der Erfindung ist jedenfalls, dass jenseits und unterhalb der Sperrnoppen ein geschützter Verpackungsraum gebildet ist, und dass der oberhalb der Sperrnoppen befindliche Bereich des Verpackungskörpers in beliebiger Weise abgedichtet verschlossen werden kann, wobei insbesondere eine luftdichte Versiegelung, zum Beispiel durch Verschweißung bevorzugt wird.

Nachdem es sich um eine Verpackung für sterilisierbare Gegenstände handelt, werden solche Verpackungskörper bevorzugt im medizinischen Bereich eingesetzt und die zu verpackenden Gegenstände sind bevorzugt medizinische, sterilisierbare Gegenstände, wie zum Beispiel Schrauben, Noppen, Muttern und Stifte und dergleichen mehr. Die Sterilisierung erfolgt bevorzugt an der mit Verpackungsgegenständen befüllten und dicht verschlossenen Verpackung im Wege einer Strahlungssterilisierung.

In einer anderen bevorzugten Ausführung ist vorgesehen, dass die elastisch aufweitbare Einzelverpackung mit Inhalt in eine luftdichte Beutelverpackung gesteckt wird und danach sterilisiert wird.

Der Verpackungskörper soll gut handbar sein. Er soll bevorzugt mit den Fingern einer Hand betätigt werden. Daher liegen die bevorzugten Dimensionen der Verpackung im Bereich einer Länge von z. B. 40 mm bis zu einer Länge von 400 mm. Eine bevorzugte Breite ist etwa 26 mm bis zu einer bevorzugten Breite von etwa 200 mm.

Es handelt sich hierbei lediglich um bevorzugte Größenabmessungen, welche den Erfindungsbereich der Erfindung nicht beschränken.

Charakteristikum der erfindungsgemäßen Verpackung ist ferner, dass es sich um einen aus Kunststoff bestehenden, vorzugsweise transparenten - oder auch leicht eingefärbten - elastisch verformbaren Flachkörper handelt, das heißt, um einen Körper, der aus zwei zueinander parallelen Wänden besteht, nämlich einer Rückwand und einer Vorderwand, und die beiden Wände parallel zueinander sind und seitlich durch schmalere Seitenwände in sich verbunden sind, wodurch sich insgesamt ein etwa flacher, etwa taschenförmiger, im Querschnitt etwa rechteckförmiger Körper ergibt.

Eine bevorzugte Form des Verpackungskörpers ist eine Rechteckform oder eine an die Rechteckform angenäherte Form mit abgerundeten Ecken.

Eine andere Ausgestaltung sieht vor, dass die zueinander parallelen Vorder- und Rückwände eine leicht bauchige (konvexe) Form haben. Ebenso können die Seitenwände eine konvexe Formgebung haben.

Die Rechteckform hat den Vorteil, dass die Verpackung gegen unbeabsichtigtes Wegrollen von einer Lagerfläche geschützt ist.

Die Betätigung erfolgt durch Fingerdruck auf einander gegenüberliegende Griffrippen, die im Bereich des oberen Verschlussteils angeordnet sind. Die Betätigungskraft entspricht etwa der Fingerkraft, die notwendig ist, um eine Wäscheklammer zu öffnen.

Dabei wird die etwa flache, ovale Querschnittsform des Verpackungskörpers im Halsbereich in eine angenäherte Rundform übergeführt. Dadurch weitet sich die vorher ovale Öffnung im oberen Bereich des Verschlussteils auf und die im unteren Bereich des Verschlussteils angeordneten, zueinander fluchtenden Sperrnoppen, die zwischen sich den Verschlussspalt bilden, entfernen sich voneinander, wodurch sich der Verschlussspalt öffnet und ein Gegenstand aus dem unteren Verpackungsraum durch den geöffneten Verschlussspalt entnommen oder eingeführt werden kann.

Bei größeren Verpackungskörpern ist vorgesehen, dass sowohl an der Vorderwand als auch an der Rückwand jeweils paarweise einander gegenüberliegende Sperrnoppen angeordnet sind. Das heißt die Sperrnoppen sind paarweise auf der Vorderwand und paarweise auf der Rückwand angeordnet und fluchten in einer horizontalen Linie zueinander und bilden zwischen sich den durch Fingerdruck oder - bei sehr großen Verpackungen - durch Maschinendruck aufweitbaren Verschlussspalt.

Der Verpackungskörper ist bevorzugt in seinem oberen Halsbereich derart elastisch ausgebildet, dass die Griffrippen durch Fingerdruck gegeneinandergepresst werden können, um so eine Aufweitung der umlaufenden Öffnungsrandes zu erreichen. Dadurch ergeben sich eine großflächige Betätigungsflächen, die auch mit einer - einen Handschuh tragenden - Hand leicht betätigt werden können.

Hierauf ist die Erfindung nicht beschränkt. In einer anderen Ausgestaltung kann es bei größeren Verpackungen vorgesehen sein, dass zur Befüllung oder zur Entnahme von Verpackungsgegenständen auch Maschinenhilfe zur elastischen Verformung des Halsbereichs in Anspruch genommen wird.

Bei kleineren Verpackungskörpern reicht es allerdings aus, nur ein Paar von gegenüberliegende Sperrnoppen im unteren Teil des Halsbereichs anzuordnen. Auf jeder Verpackungsseite ist demnach nur eine Sperrnoppe vorgesehen. Bei größeren Verpackungen können auf jeder Verpackungsseite mehrere Sperrnoppen in gegenseitigem Abstand angeordnet sind, die in Lückeneingriff mit den auf der gegenüberliegenden Verpackungsseite angeordneten Sperrnoppen kommen, um so den Verschlussspalt zu bilden.

Die Formgebung der Sperrnoppen ist in weiten Grenzen variierbar. Es kann vorgesehen sein, dass die Sperrnoppen den Verschlussspalt zwar in der Verschlussstellung sicher begrenzen, andererseits aber ein Herausschütteln oder Herausgleiten des verpackenden Gegenstandes nicht behindern. Daher können die in Lückeneingriff stehenden Sperrnoppen einen gegenseitigen Abstand einnehmen. **In** dieser Variante besteht deshalb kein reibschlüssiger gegenseitiger Kontakt zwischen den Sperrnoppen.

**In** einer anderen Variante können die in Lückeneingriff stehenden Sperrnoppen einen gegenseitigen reibschlüssigen Kontakt haben.

Ebenso wird es bevorzugt, wenn die paarweise angeordneten Sperrnoppen einen gegenseitigen Abstand zwischen sich bilden, weil beim Zusammendrücken eines solchen Verpackungskörpers eine Knickung des Materials zwischen den Sperrnoppen im Bereich dieses Zwischenabstandes erfolgt und dadurch eine verbessere Aufweitung der Öffnung und des Verschlussspaltes möglich ist.

Bei einer anderen Ausgestaltung der Erfindung kann es vorgesehen sein, dass die paarweise jeweils auf der Vorderwand und der Rückwand angeordneten Sperrnoppen miteinander verbunden sind, das heißt der mittige Abstand zwischen den Sperrnoppen - der vorher den Knickbereich bildete - entfällt dann. Damit ist dafür gesorgt, dass die beiden paarweise angeordneten Sperrnoppen zu jeweils einer einzigen horizontalen langgestreckten Sperrnoppe auf der Vorderwand und der Rückwand verschmelzen, wobei immer vorausgesetzt wird, dass die Sperrnoppen auf der Vorder- und der Rückwand zueinander fluchten.

Die Erfindung ist im Übrigen nicht darauf angewiesen, dass die Sperrnoppen sowohl auf der Vorderwand als auch auf der Rückwand angeordnet sind. Es kann in einer davon abweichenden Ausgestaltung vorgesehen sein, dass entweder nur an der Innenseite der Vorderwand oder nur an der Innenseite der Rückwand einzelne oder paarweise angeordnete Sperrnoppen vorhanden sind, die einen Verschlussspalt zur jeweils glatten Innenseite der gegenüberliegenden Wand bilden, wodurch der Verschlussspalt dann nicht mehr mittig in dem Verpackungskörper angeordnet ist, sondern seitlich versetzt ist.

In einer weiteren Ausgestaltung der Erfindung ist vorgesehen, dass die auf der Vorderwand und der Rückwand einander gegenüberliegend angeordneten Sperrnoppen nicht zueinander fluchten, sondern auf einer horizontalen Linie seitlich gegeneinander versetzt sind, sodass sie eine in sich ineinandergreifende Zahnreihe bilden.

Die auf Abstand an der Vorderwand angeordneten Sperrnoppen greifen dann in der Art eines Zahneingriffes in die Zwischenräume der auf der Rückwand angeordneten Sperrnoppen ein und bilden so einen verzahnten, labyrinthartigen Verschlusspalt, der dadurch eine besondere Verschlusscharakteristik hat. Der Verzahnungseingriff kann in einer ersten Ausführung ein gegenseitiges Spiel zwischen den ineinandergreifenden Sperrnoppen erlauben. In einer zweiten Ausführung kann der Verzahnungseingriff auch reibschlüssig sein, das heißt, die ineinandergreifenden "Zähne" der Sperrnoppen bilden eine zusätzliche Verschlusskraft aus, deren Betrag über die elastische Rückstellkraft des Kunststoffs im Halsbereich hinausgeht.

Die paarweise gegeneinander gerichteten, einen gegenseitigen Zahneingriff bildenden Sperrnoppen müssen nicht nur paarweise vorhanden sein, sondern es reicht in einer anderen Variante der Erfindung aus, dass eine Sperrnoppe an der Vorderseite und eine gegenüberliegende Sperrnoppe an der Rückseite angeordnet ist und die beiden Sperrnoppen aber nicht zueinander fluchten, sondern seitlich zueinander versetzt sind, um so ebenfalls einen Zahneingriff zu ermöglichen und damit einen abgeknickten Verschlussspalt bilden.

Während also die ersten Ausführungsformen, die bevorzugt in den Zeichnungen beschrieben sind, einen horizontalen, gerade durchlaufenden Verschlusspalt beschreiben, sind in den soeben beschriebenen Varianten die Sperrnoppen so angeordnet, dass sie einen gegenseitigen Zahneingriff bilden, um so nicht einen langgestreckten ebenen Verschlussspalt zu bilden, sondern einen gezackten, ineinandergreifenden Verschlussspalt.

Bei allen Ausführungsformen kann es zur weiteren Verbesserung der Lagensicherung vorgesehen sein, dass der untere Teil des zu haltenden Gegenstandes an verpackungsseitigen Anschlagnoppen aufsteht, um so eine Begrenzung der Verschiebungslage des Gegenstandes im Verpackungskörper zu vermeiden. Solche Anschlagnoppen können bevorzugt plane Anschlagflächen für die untere Stirnseite des Gegenstandes bilden. In einer anderen Ausgestaltung können sie auch mit Einführschrägen ausgebildet sein

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, könnten als erfindungswesentlich beansprucht werden, soweit sie einzeln oder in Kombination gegenüber dem Stand der Technik neu sind. Die Verwendung der Begriffe "wesentlich" oder "erfindungsgemäß" oder "erfindungswesentlich" ist subjektiv und impliziert nicht, dass die so benannten Merkmale zwangsläufig Bestandteil eines oder mehrerer Patentansprüche sein müssen.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Ansicht einer ersten Ausführung eines Verpackungskörpers
- Figur 1a:: Die Draufsicht auf die Öffnung des Verpackungskörpers nach Figur 1
- Figur 2:: Ein Längsschnitt durch den Verpackungskörper nach der Schnittlinie A-A in Figur 1
- Figur 3:: Ansicht einer zweiten Ausführung eines Verpackungskörpers
- Figur 4:: Schnitt durch den Verpackungskörper nach der Schnittlinie B-B in Figur 3
- Figur 4a:: Draufsicht auf den zweiten Verpackungskörper in Richtung seiner Öffnung
- Figur 5:: Ansicht einer dritten Ausführung eines Verpackungskörpers Seitenansicht des Verpackungskörpers nach Figur 1 und 2
- Figur 6:: Draufsicht auf den dritten Verpackungskörper in Richtung seiner Öffnung
- Figur 7:: Ansicht des ersten Verpackungskörpers nach Figur 1 in aufgeweitetem Zustand
- Figur 8:: Die Draufsicht auf die verformte Öffnung des Verpackungskörpers nach Figur 7
- Figur 9:: Ansicht des zweiten Verpackungskörpers nach Figur 3 in aufgeweitetem Zustand
- Figur 10:: Die Draufsicht auf die verformte Öffnung des Verpackungskörpers nach Figur 9
- Figur 11:: Ansicht des dritten Verpackungskörpers nach Figur 3 in aufgeweitetem Zustand
- Figur 12:: Die Draufsicht auf die verformte Öffnung des Verpackungskörpers nach Figur 11
- Figur 13:: Ansicht einer ersten Variante des dritten Verpackungskörpers nach Figur 5 in unverformtem Zustand
- Figur 14:: Draufsicht auf den dritten Verpackungskörper nach Figur 13 in Richtung seiner Öffnung
- Figur 15:: Ansicht einer zweiten Variante des dritten Verpackungskörpers nach Figur 5 in unverformtem Zustand
- Figur 16:: Draufsicht auf den dritten Verpackungskörper nach Figur 15 in Richtung seiner Öffnung
- Figur 17:: Ansicht einer dritten Variante des dritten Verpackungskörpers nach Figur 5 in unverformtem Zustand
- Figur 18:: Draufsicht auf den dritten Verpackungskörper nach Figur 17 in Richtung seiner Öffnung
- Figur 19:: Ansicht des dritten Verpackungskörpers nach Figur 13 in verformtem Zustand
- Figur 20:: Draufsicht auf den Verpackungskörper nach Figur 19 in Richtung seiner Öffnung in verformtem Zustand
- Figur 21:: Ansicht des Verpackungskörpers nach Figur 15 in verformtem Zustand
- Figur 22:: Draufsicht auf den Verpackungskörper nach Figur 21 in Richtung seiner Öffnung in verformtem Zustand
- Figur 23:: Ansicht des Verpackungskörpers nach Figur 17 in verformtem Zustand
- Figur 24:: Draufsicht auf den Verpackungskörper nach Figur 23 in Richtung seiner Öffnung in verformtem Zustand
- Figur 25:: Ansicht einer vierten Variante des Verpackungskörpers nach Figur 9 in unverformtem Zustand
- Figur 26:: Draufsicht auf den Verpackungskörper nach Figur 25 in Richtung seiner Öffnung in unverformtem Zustand
- Figur 27:: Ansicht der vierten Variante des Verpackungskörpers nach Figur 25 in verformtem Zustand
- Figur 28:: Draufsicht auf den Verpackungskörper nach Figur 27 in Richtung seiner Öffnung in verformtem Zustand
- Figur 29:: Draufsicht auf den Verpackungskörper nach den Figuren 25-28 mit einem verpackten Gegenstand
- Figur 30:: Draufsicht auf die Öffnung des Verpackungskörpers nach Figur 29
- Figur 31:: Schnitt durch den Verpackungskörper gemäß Schnittlinie B-B in Fig. 30
- Figuren 32-34:: Schrittweise Darstellung der Beladung oder Entladung des Verpackungskörpers mit einem Gegenstand
- Figur 35:: Schnitt durch den Verpackungskörper nach Fig. 1 oder 7 im verformten Zustand bei der Entnahme eines Gegenstandes
- Figur 36:: Die Draufsicht auf die Entnahmeöffnung
- Figur 37:: die Seitenansicht des Verpackungsköpers nach Fig. 35
- Figur 38-40:: Schrittweise Darstellung der Entnahme des Gegenstandes aus dem Verpackungskörper nach den Fig. 35-37
- Figur 41:: Schnitt durch einen beladenen Verpackungskörper nach Fig. 15 und 21 mit einem anderen Verpackungsgegenstand
- Figur 42:: Draufsicht auf die Mündung des Verpackungskörpers nach Fig. 41 und 43
- Figur 44-46:: Schrittweise Darstellung der Entnahme des Gegenstandes aus dem Verpackungskörper nach den Fig. 41-43

Der Verpackungskörper 1 nach der Erfindung hat in sämtlichen Zeichnungen eine im Wesentlichen flache langgestreckte Körperform, das heißt, er ist im Querschnitt etwa rechteckförmig, denn er besteht aus einer flachen Vorderwand 2, einer hierzu parallelen Rückwand 4 und zwei sich werkstoffeinstückig anschließenden Seitenwänden 3.

Der gesamte Verpackungskörper 1 ist bevorzugt in einem Stück im Hohlblasverfahren hergestellt und besteht aus einem elastischen Kunststoff, bevorzugt einem TPU-Kunststoff. Daher ist er sterilisierbar, schweißbar und abriebfest. Er ist bevorzugt transparent oder leicht eingefärbt und dauerelastisch. Er bildet eine flache Verpackungstasche. Er hat bevorzugt eine Härte im Bereich von 45 bis 95 shore-A und dabei besonders bevorzugt eine Härte von 85 shore-A +- 5.

Die bevorzugten Abmessungen wurden bereits schon im allgemeinen Beschreibungsteil erwähnt, wobei der Verpackungskörper nach der Erfindung für die Verpackung relativ kleiner Gegenstände geeignet sein soll, wie sie im medizinischen Bereich vorkommen, wobei unter dem Begriff "kleine Gegenstände" auch Organersatzteile gehören, wie zum Beispiel künstliche Kniegelenke, Schultergelenke, Knochennägel, Hüftgelenke, Knochensägeblätter und/oder Kleinteile, wie zum Beispiel Schrauben, Muttern, Stifte, Platten und dergleichen mehr. Die Art und Anordnung der im Innenraum der Verpackung angeordneten Mittel zur Lagensicherung und Führung des verpackten Gegenstandes, sowie die lagenbegrenzenden unteren Anschlagnoppen sind an den Körper des zu verpackenden Gegenstandes angepasst. Aus diesem Grund sind in den nachfolgenden Zeichnungen nur einige Beispiele für die Verpackung von unterschiedlichen Gegenständen angegeben.

Die in den Zeichnungen dargestellten Ausführungsbeispiele zeigen bevorzugte tatsächliche Abmessungen eines Verpackungskörpers im Abbildungsmaßstab 1:1 zur sterilen Verpackung von einzelnen Schrauben, Muttern, Stifte, Platten und dergleichen.

Der die Gegenstände aufnehmende Verpackungsraum 27 ist langgestreckt und bodenseitig durch eine abgerundetes Bodenteil 5 abgeschlossen. Der Verpackungsraum 27 und das Bodenteil 5 sind jedoch nur geringfügig elastisch zusammendrückbar, denn die Zusammendrückbarkeit wird durch die im Verpackungsraum 27 angeordnete Führungsvorrichtung 22 beschränkt.

Diese elastische Verformbarkeit des Halsbereiches 8 erleichtert die Entnahme von im Verpackungsraum 27 gelagerten Gegenständen 17.

Der Übergang vom im Querschnitt ovalen Verpackungsraum 27 in den sich oben anschließenden ovalen Halsbereich 8 eines Verschlussteils 6 erfolgt über eine bogenförmige konvexe Kontur. Der Halsbereich 8 ist deshalb im Querschnitt gegenüber dem Verpackungsraum 27 in seiner Breite verringert. Er bildet eine obere Öffnung 9, die durch elastische Verformung in eine der Rundform angenäherte Öffnung 9' übergeführt werden kann.

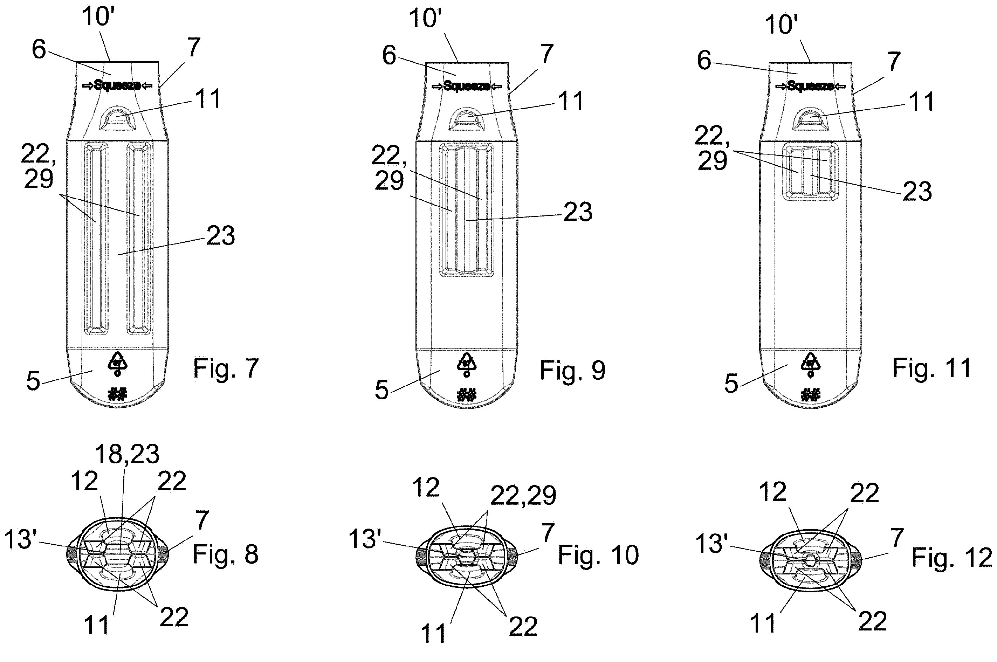

Die unverformte ovale Öffnung 9 ist durch einen umlaufenden Rand 10 definiert und im Bereich der Seitenwände 3 sind im Bereich des Verschlussteils 6 zueinander fluchtend gegenüberliegende Griffrippen 7 angeordnet, die über Fingerdruck oder - bei automatischen Verpackungsmaschinen mit Maschinenhilfe - gegeneinander gequetscht werden können. Die sich hieraus ergebende Formgebung ist in den folgenden Figuren 7 ff. beschrieben.

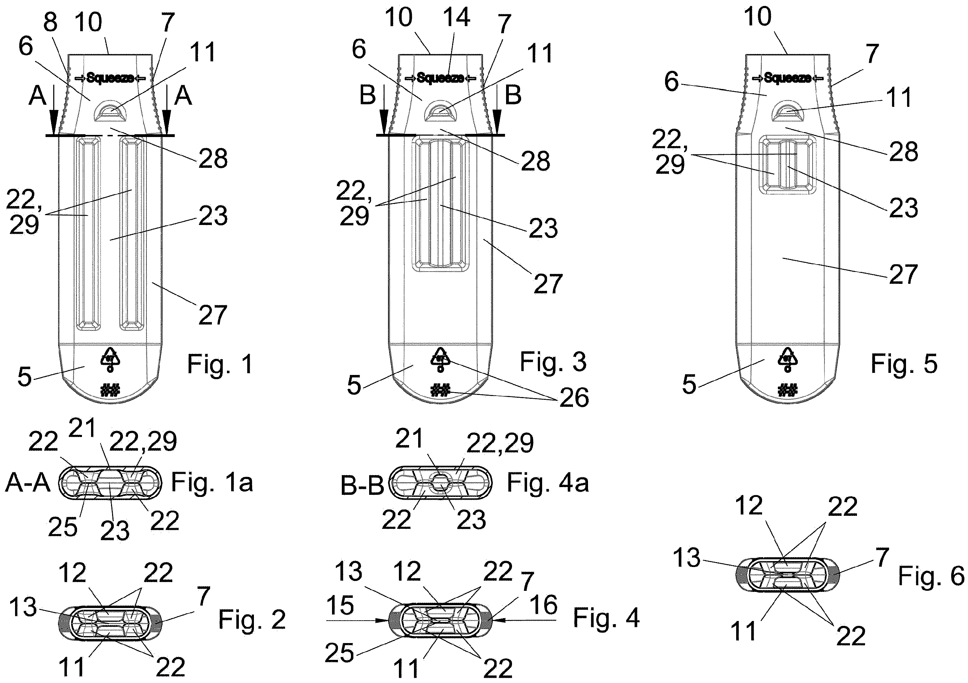

Wichtig ist, dass im unteren Bereich des Verschlussteils 6 und im Übergang zum Verpackungsraum 27 eine Sperrmechanik angeordnet ist, die im gezeigten Ausführungsbeispiel aus paarweise gegenüberliegenden Sperrnoppen 11, 12 gebildet ist, die zueinander fluchtend angeordnet sind, wobei ein Paar der Sperrnoppen 11 auf der Innenseite der Vorderwand 2 und ein gleiches Paar von Sperrnoppen 12 auf der Innenseite der Rückwand 4 angeordnet sind und die paarweise angeordneten Sperrnoppen 11, 12 fluchtend gegenüberliegen, wie dies aus den Figuren 1a, 2, 4, 4a, 6 erkennbar ist. Sie bilden im geschlossenen Zustand demnach einen in horizontaler Richtung verlaufenden Verschlussspalt 13, der im geschlossenen Zustand eine bevorzugte Weite im Bereich von 0 mm bis 1 mm haben kann.

In der allgemeinen Beschreibung wurde bereits schon darauf hingewiesen, dass es nicht lösungsnotwendig ist, dass sich die paarweise gegenüberliegend angeordneten Sperrnoppen 11, 12 gegenseitig berühren. Es kann aber in einer anderen Ausführungsform vorgesehen sein, dass die Sperrnoppen 11, 12 auf Lücke zueinander versetzt sind, um so einen verzahnten abgedichteten oder nicht abgedichteten Eingriff miteinander zu bilden.

Ebenso wurde in der allgemeinen Beschreibung schon erwähnt, dass anstatt der Anordnung von paarweisen Sperrnoppen 11, 12 auch Einzelsperrnoppen 11, 12 vorgesehen sein können.

Es können aber auch mehr als zwei Sperrnoppen jeweils an einer Innenseite der Vorderwand oder einer Innenseite der Rückwand angeordnet sein.

Demnach ist die Anzahl der Sperrnoppen, die an der Vorderwand 2 und auf der Rückwand 4 angeordnet sind, nicht beschränkt. Es können auch mehr als zwei vorhanden sein.

Es wird bevorzugt, wenn im Bereich des Verschlussteils 6 eine Beschriftung 14 angeordnet ist, die darauf hinweist, dass die Griffrippen 7 durch Fingerdruck gegeneinander in den Pfeilrichtungen 15, 16 (siehe Fig. 4) komprimiert werden können, um so die Verpackung in einen geöffneten Zustand zu überführen.

Die Figuren 2-6 zeigen weitere Einzelheiten der Konstruktion, wobei aus Figur 2 noch erkennbar ist, dass zwischen den paarweise angeordneten Sperrnoppen 11, 12 jeweils ein Abstand vorhanden ist, der als Knickbereich 21 wirkt. Die Anordnung des Knickbereiches 21 ermöglicht, dass wenn die Griffrippen 7 gegeneinander komprimiert werden, das Material sich im Bereich des Knickbereiches 21 konvex ausbaucht, um so eine Verringerung der Kompressionskraft bei Öffnung des Verschlussteils 6 zu ermöglichen. Damit wird die durch Fingerdruck bewirkte Zusammendrückungskraft verringert und gleichzeitig die Weite des Verschlussspaltes 15 vergrößert. Die Zusammendrückungskraft entspricht in allen Fällen - unabhängig vom Vorhandensein eines Knickbereiches 21 oder nicht - bevorzugt der Kraft, die aufgewendet werden muss, um eine Wäscheklammer zu öffnen.

In einer anderen Ausgestaltung kann es vorgesehen sein, dass die auf der Vorder- und der Rückwand paarweise angeordneten Sperrnoppen 11, 12 jeweils zu einer einzigen horizontalen Sperrnoppe umgeformt sind.

Bei der Beschreibung des vorliegenden Verpackungskörpers ist es gleichgültig, welche Formgebung die Verpackungsgegenstände haben, wie nachfolgend gezeigt wird. Das heißt, die Verpackungsgegenstände können eine Länge haben, sodass sie gerade im Verpackungsraum 27 oben an der Unterseite der Sperrnoppen 11, 12 anstoßen und unten in den Bodenteil 5 hineinragen. Sie können aber auch wesentlich kürzer sein, sodass die Verpackung die Charakteristik einer taschenförmige Einzelverpackung hat.

Es können somit eine Vielzahl von einzelnen Gegenständen 17 über den geöffneten Verschlussspalt 13' in den Verpackungsraum 25 hineingeführt werden, um dann bei geöffnetem Verschlusspalt 13' wieder entnommen zu werden.

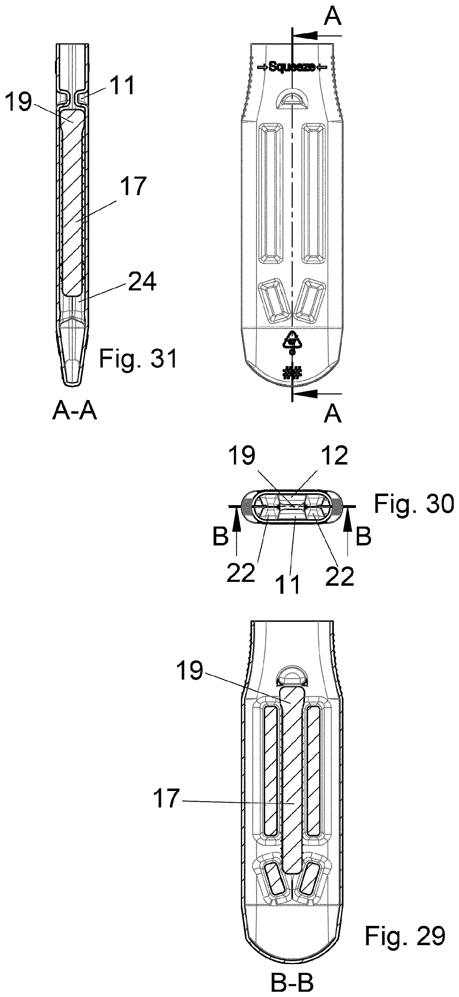

Die Figur 7 bis 12 zeigt den geöffneten Verpackungskörper 1, wobei vorausgesetzt wird, dass in den Pfeilrichtungen 15, 16 mit Fingerdruck oder mit maschineller Hilfe ein gegenseitiger Druck auf die Griffrippen 7 ausgeübt wird und sich die Öffnung 9 in eine etwa rundprofilierte - oder vergrößerte ovale - Öffnung 9' umformt. Dabei öffnet sich auch der Verschlussspalt 13 in den Verschlussspalt 13', weil sich die Sperrnoppen 11, 12 voneinander entfernen und den im Querschnitt vergrößerten und geöffneten Verschlussspalt 13' bilden. Gemäß den Figur 29 bis 46 können dann durch den geöffneten Verschlussspalt 13' hindurch die zu verpackenden Gegenstände 17 in den Verpackungsraum 27 eingeführt werden.

Die Figuren 1a und 2 zeigen, dass die Sperrnoppen 11, 12 in bestimmter Weise profiliert sind, das heißt, sie haben bevorzugt seitliche Einführschrägen, sodass ein besonders einfaches Einführen eines zu verpackenden Gegenstandes über die Einführschrägen möglich ist.

Solche Einführschrägen oder auch unten angeordnete Ausführschrägen können auch entfallen.

Der Vergleich der Figuren 1, 3 und 5 zeigt, dass bei gleicher Verpackungsgröße die im Innenraum angeordnete Führungsvorrichtung 22 eine unterschiedliche axiale Länge aufweisen kann. Die Länge der Führungsvorrichtung 22 richtet sich nach der Art des zu haltenden Gegenstandes und nach den Eigenschaften, die dieser Führungsvorrichtung zugeordnet werden sollen.

Jede Führungsvorrichtung 22 besteht aus 2 seitlichen, rillenförmigen Begrenzungsrippen 29, die jeweils im gegenseitigen Abstand parallel zueinander in der Vorder- und Rückwand 2, 4 eingekerbt sind und in ihrem gegenseitigen Berührungsbereich im Verpackungsraum 27 über den Verbindungsbereich 25 miteinander verbunden sind. Sie verbinden deshalb die Vorderwand 2 mit der Rückwand 4. Demnach handelt es sich um stabile, biegungssteife Begrenzungsrippen 29, welche die Länge der Führungsvorrichtung 22 bestimmen und die zwischen sich einen etwa rundprofilierten Führungskanal 23 bilden, der zur Aufnahme des unteren Teils des Gegenstandes 17 geeignet ist. Damit wird der zu verpackende Gegenstand 17 in einer besonderen Lagensicherung im Führungskanal 23 im Bereich der verpackungsraumseitigen Führungsvorrichtung 22 verkantungssicher gehalten.

Wie im allgemeinen Beschreibungsteil erwähnt, kann dieser Führungskanal 23 auch in bestimmter Weise profiliert sein, um sich an das Profil des zu haltenden Gegenstandes 17 in diesem Bereich anzupassen. Wenn eine solche Profilierung gegeben ist, zum Beispiel eine ovale, rechteckige oder polygonale Profilform aufweist, wird der zu haltende Gegenstand gegen Verdrehung gesichert im Führungskanal 23 gehalten und kann sie nicht mehr um seine eigene Längsachse drehen.

Der Führungskanal 23 ist bevorzugt im Längsmittenbereich der Führungsvorrichtung 22 angeordnet.

Die starre Verbindung der axialen Einkerbungen der Führungsvorrichtung sorgt dafür, dass sie nicht gebogen oder in anderer Weise verformt wird, selbst wenn der Verpackungskörper 1 zusammengequetscht wird.

Die Figuren 2, 4, 4a und 6 zeigen jeweils die Ansicht auf die unverformte Öffnung 9 mit dem umlaufenden Rand 10. Sie sind bei den Verpackungskörpern 1 nach den Figuren 1, 3 und 5 gleich ausgebildet.

Die Figuren 7, 9, 11 zeigen die gleichen Verpackungskörper 1 nach den Figuren 1, 3 und 5, jedoch in verformtem Zustand des Halsbereiches 8.

Aus den Darstellungen der Figuren 8, 10 und 12 ist entnehmbar, dass nur der Verschlussspalt 13 in den geöffneten Verschluss bald 13 Strich übergeführt wurde und sich daher die Sperrnoppen 11,12 voneinander entfernt haben und dadurch der Führungskanal 23 offen ist und nicht mehr durch die Sperrnoppen 11,12 verschlossen ist.

Die Sperrnoppen 11, 12 bilden somit zwischen sich einen Durchtrittskanal 18, der axial fluchtend zu dem Führungskanal 23 der Führungsvorrichtung ausgebildet ist.

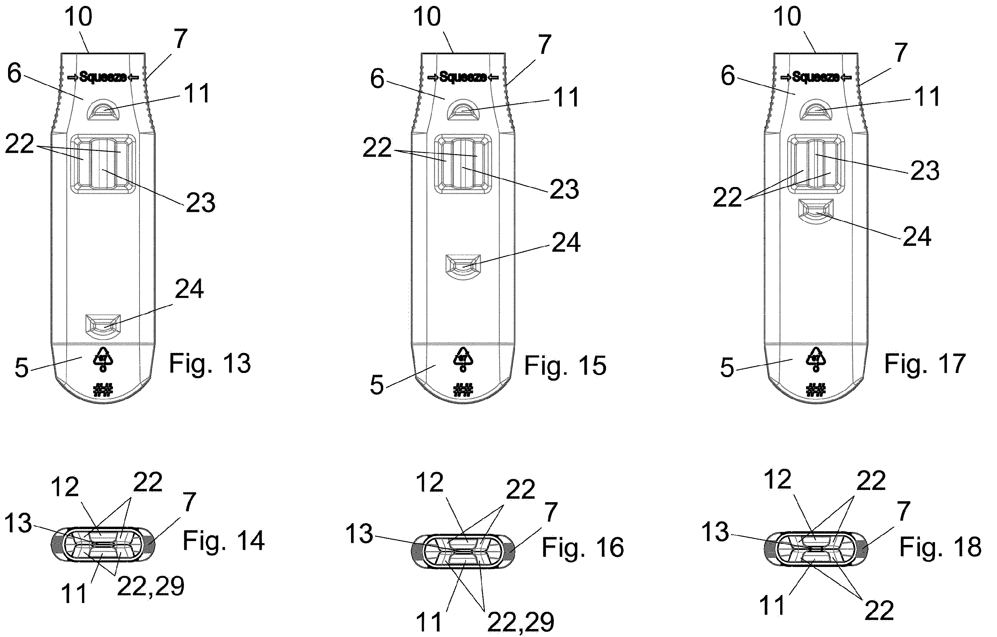

Die Figuren 13, 15 und 17 zeigen weitere Ausführungsformen eines Verpackungskörpers, welche sich von den vorher beschriebenen Ausführungsformen lediglich dadurch unterscheiden, dass im Bereich an der Unterseite des Verpackungsraums 27 ein oder mehrere Anschlagnoppen 24 angeordnet sind, die zum Anschlag der unteren Stirnseite des zu haltenden Gegenstandes vorgesehen sind.

Die Anschlagnoppen 24 können eine ebene Aufstandsfläche für den dort aufsitzenden Gegenstand ausbilden. In anderen Ausgestaltungen können sie jedoch auch der Profilform des Gegenstandes angepasst sein und damit eine Verdrehsicherung bilden oder ein oder mehrere Einführschräge aufweisen.

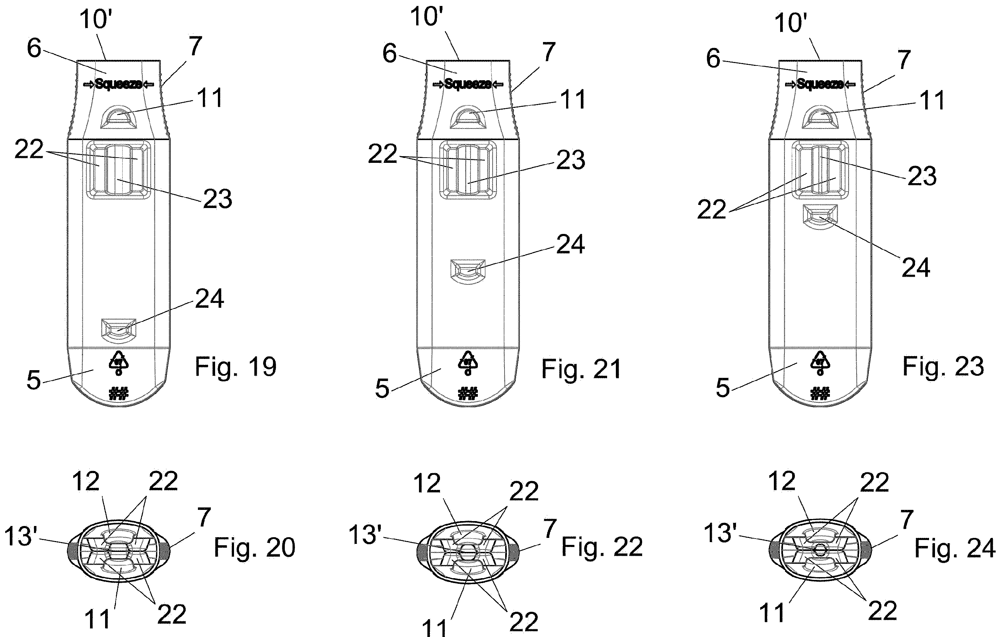

Die Figuren 19, 21, 23 zeigen den elastisch verformten Verpackungskörper nach den Figuren 13, 15 und 17. Daher zeigen die Figuren 20, 22 und 24 das Öffnungsbild des Verpackungskörpers mit Darstellung der elastisch aufgeweiteten Öffnung 9'.

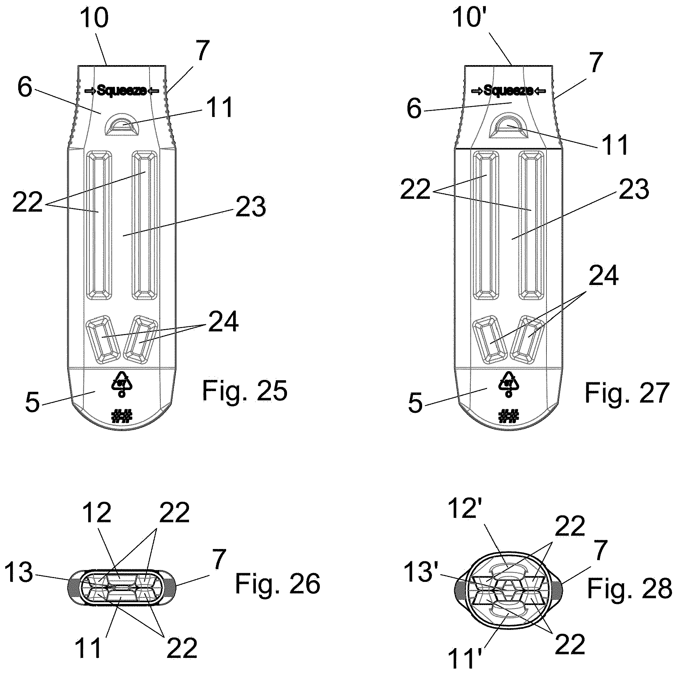

Das Ausführungsbeispiel nach den Figuren 25 bis 28 zeigt eine weitere Variante eines Verpackungskörpers, die sich von den vorhergehenden Ausführungsbeispielen nur dadurch unterscheidet, dass im Verpackungsraum 27 2 im Abstand zueinander angeordnete Anschlagnoppen 24 angeordnet sind, die schräg gegeneinander gerichtet sind und so eine nach oben geöffnete Einführschräge für einen dort zu haltenden Gegenstand bilden. Damit erfolgt eine noch eine bessere Zentrierung des zu haltenden Gegenstandes 17, die dann nicht nur im Führungskanal 23 stattfindet, sondern darüber hinaus auch noch im Bereich der bodenseitigen Anschlagnoppen 24. Die Figuren 26 und 28 zeigen die Ansicht auf die unverformte und die verformte Öffnung 9.

Die Figuren 29 bis 34 zeigen anhand des Ausführungsbeispiels nach den Figuren 25-28 ein bevorzugtes Beispiel, wie ein zu verpackende Gegenstand 17 im Verpackungsraum 27 einer in der Figuren 25-28 gezeigten Verpackung gehalten ist. Die Figur 31 zeigt, dass sich der Schraubenkopf 19 des Gegenstandes 17 an der Unterseite der Sperrnoppen 11,12 in einem Freiraum 28 anliegt und mit seinem bodenseitigen Ende an den vorher beschriebenen Anschlagnoppen 24 aufsitzt. Dies ist auch aus den Figuren 29 und 30 zu entnehmen. Es handelt sich damit um eine lagengesicherte und gegen Vibrationen geschützte Halterung eines Gegenstandes 17, dessen stirnseitiges, unteres Ende in die Anschlagschrägen der Anschlagnoppen 24 eingreift und dort verschiebungsgesichert gehalten ist. Eine solche Halterung hat den Vorteil, dass sich der Gegenstand 17 im Verpackungsraum 27 auch bei Einwirkungen von Vibrationskräften nicht mehr bewegen kann und nicht an seinen verpackungsseitigen Anlageflächen in unbeabsichtigter Weise Kunststoffmaterial abschabt und damit kontaminiert wird.

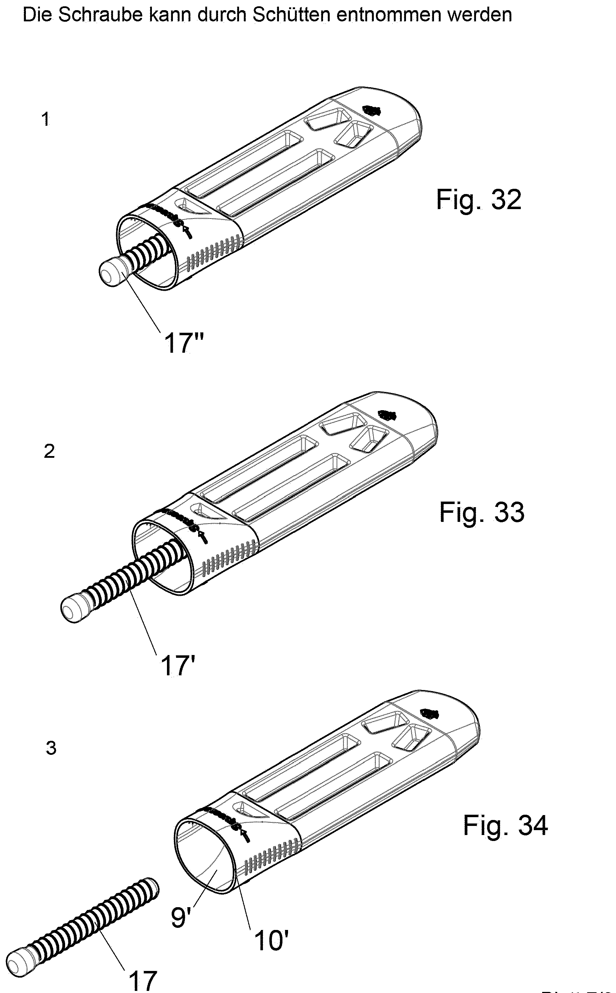

Die Figuren 32-34 zeigen das schrittweise Einführen oder Entnehmen eines Gegenstandes 17, 17' und 17" aus einer Verpackung nach den Figuren 25-31. Der als Schraube ausgebildete Gegenstand 17 kann bei zusammengequetschten Öffnung 9' auch durch Schütten entnommen werden.

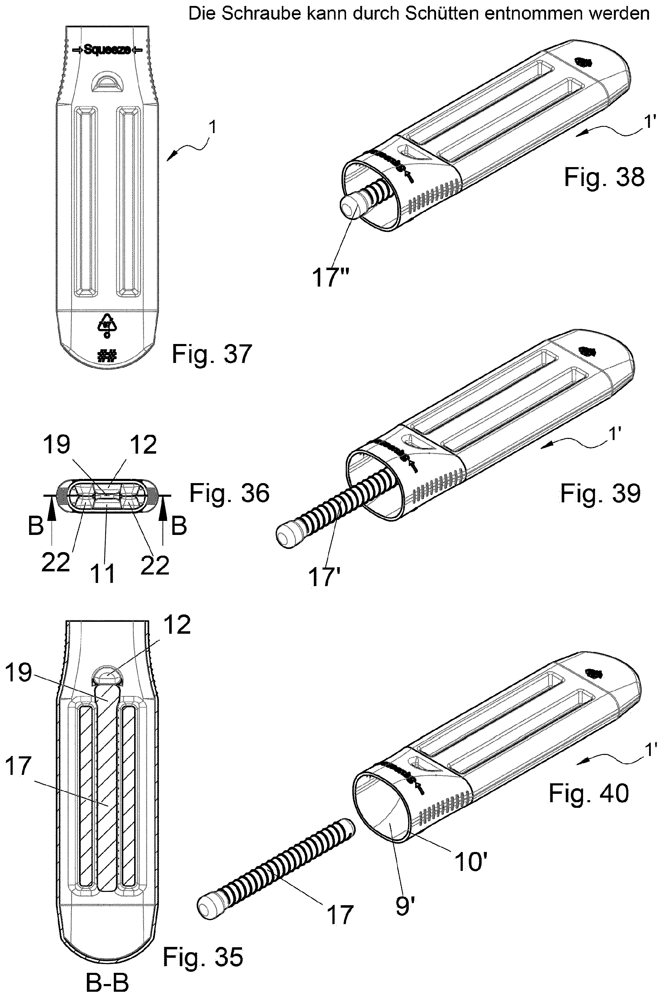

Die Figuren 35 bis 40 zeigen den gleichen Vorgang, wie in den Figuren 32-34 beschrieben mit einem Verpackungskörper 1 nach einer der Ausführungen nach den Figuren 1 und 7. Im Unterschied zur Ausführung nach den Figuren 29-31 wird der Schraubenkopf 19 allein verschiebungsgesichert mit seiner Oberseite an der Unterseite der Sperrnoppen 11, 12 und mit seiner Unterseite am Einlaufbereich des Führungskanals 23. Ein unterer, bodenseitiger Anschlag entfällt.

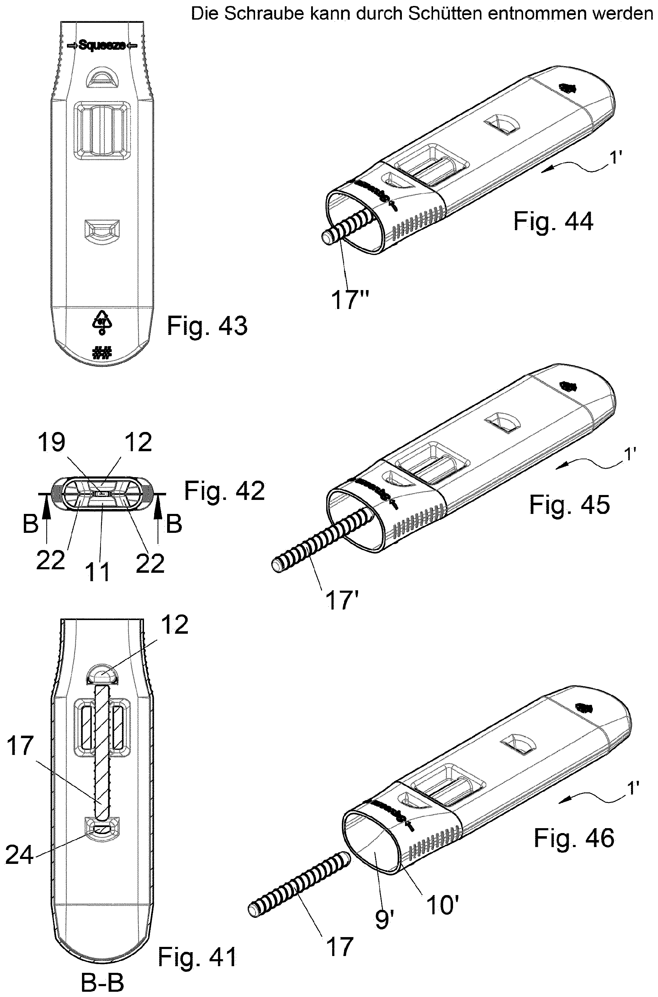

Die Figuren 41 bis 44 zeigen eine Abwandlung der Ausführung nach den Figuren 35 bis 40 mit dem Unterschied, dass das untere Ende des Gegenstandes auf einer unteren Anschlagnoppe 24 aufsitzt und statt der Schraube ein Stift vorhanden ist, der keinen Schraubenkopf 19 aufweist.

### Zeichnungslegende

- 1): Verpackungskörper
- 1'): Verpackungskörper
- 2): Vorderwand
- 3): Seitenwand
- 4): Rückwand
- 5): Bodenteil
- 6): Verschlussteil
- 7): Griffrippen
- 8): Halsbereich (von 6)
- 8'): Halsbereich (von 6)
- 9): Öffnung, 9'
- 10): Rand
- 10'): Rand
- 11): Sperrnoppe (vorne)
- 12): Sperrnoppe (hinten)
- 13): Verschlussspalt 13'
- 14): Beschriftung
- 15): Pfeilrichtung
- 16): Pfeilrichtung
- 17): Gegenstand, 17', 17"
- 18): Durchtrittskanal
- 19): Schraubenkopf
- 20) 20') 21): Knickbereich
- 22): Führungsvorrichtung
- 23): Führungskanal (mit 29)
- 24): Anschlagnoppe
- 25): Verbindungsbereich (von 22)
- 26): Beschriftungssymbol
- 27): Verpackungsraum
- 28): Freiraum
- 29): Begrenzungsrippe (für 23)

## Patentansprüche

1. Elastisch aufweitbare, sterilisierbare Einzelverpackung für langgestreckte Gegenstände (17) im medizinischen Bereich, bestehend aus einem einseitig offenen Verpackungskörper (1, 20), der einen bodenseitig geschlossenen Verpackungsraum (27) aufweist, der an seiner oberen Seite in ein elastisch aufweitbares Verschlussteil (6) übergeht, **dadurch gekennzeichnet, dass** im Übergangsbereich zwischen dem Verschlussteil (6) und dem Verpackungsraum (27) ein oder mehrere Sperrnoppen (11, 12) angeordnet sind, die den Verpackungsraum nach oben hin begrenzen und die zwischen sich einen elastisch aufweitbaren Verschlussspalt (13, 13') bilden und dass im Verpackungsraum (27) eine zum Verschlussspalt (13, 13') in vertikaler Linie fluchtende Führungsvorrichtung (22) zur lagengesicherten Führung des unteren Teils des verpackten Gegenstandes (17) angeordnet ist.

2. Einzelverpackung nach Anspruch 1, **dadurch gekennzeichnet, dass** der Verpackungskörper (1, 20) als elastisch aufweitbarer, flacher, taschenförmiger Kunststoffkörper ausgebildet ist.

3. Einzelverpackung nach Anspruch 1 oder 2, **dadurch gekennzeichnet, dass** das Verschlussteil (6) durch Fingerkraft elastisch aufweitbar ist.

4. Einzelverpackung nach einem der Ansprüche 1 bis 3, **dadurch gekennzeichnet, dass** am Verschlussteil (6) mindestens zwei auf den Schmalseiten des Verpackungskörpers (1, 20) gegenüber liegend angeordnete Griffrippen (7) angeordnet sind, die gegeneinander gerichtet elastisch verformbar sind.

5. Einzelverpackung nach Anspruch 4, **dadurch gekennzeichnet, dass** die Sperrnoppen (11, 12) zur Ausbildung des Verschlussspaltes (13) im Zwischenraum zwischen den einander gegenüberliegenden Griffrippen (7) angeordnet sind.

6. Einzelverpackung nach einem der Ansprüche 1 bis 5, **dadurch gekennzeichnet, dass** der Verpackungsraum (27) zur Aufnahme eines einzigen Gegenstandes (17) ausgebildet ist, der von der Führungsvorrichtung (22) gehalten ist und dass im axialen Abstand zwischen dem Führungskanal (23) und den Sperrnoppen (11, 12) ein Freiraum (28) vorhanden ist.

7. Einzelverpackung nach einem der Ansprüche 1 bis 6, **dadurch gekennzeichnet, dass** im Verpackungsraum (27) in fluchtender Verlängerung des Führungskanals (23) der Führungsvorrichtung (22) mindestens eine bodenseitige Anschlagnoppe (24) angeordnet ist, auf welcher der zu haltende Gegenstand (17) mit seinem unteren Ende aufsitzt.

8. Einzelverpackung nach einem der Ansprüche 1 bis 7, **dadurch gekennzeichnet, dass** die Sperrnoppen (11, 12) jeweils einzeln oder paarweise an den Innenseiten der Vorderwand (2) und der Rückwand (4) angeordnet sind und in horizontaler Richtung zueinander fluchtend gegenüberliegen und den elastisch aufweitbaren Verschlussspalt (13) bilden.

9. Einzelverpackung nach einem der Ansprüche 1 bis 8, **dadurch gekennzeichnet, dass** die Führungsvorrichtung (22) aus zwei zueinander parallelen und einen gegenseitigen Abstand zueinander ausbildenden Begrenzungsrippen (23) besteht, die zwischen sich einen hülsenartigen Führungskanal (23) zur Längsführung eines Teils des Gegenstandes (17) im Verpackungsraum (27) bilden.

10. Einzelverpackung nach Anspruch 9, **dadurch gekennzeichnet, dass** die Begrenzungsrippen (23) jeweils die Vorderwand (2) und die Rückwand (4) des Verpackungsköpers (1) auf ihrer gesamten Länge miteinander verbinden.

11. Einzelverpackung nach einem der Ansprüche 1 bis 10, **dadurch gekennzeichnet, dass** der Übergang vom ovalen Verpackungsraum (25) in den sich oben anschließenden ovalen Halsbereich (8) des Verschlussteils (6) über eine bogenförmige konvexe Kontur erfolgt, sodass der Halsbereich (8) im Querschnitt gegenüber dem Verpackungsraum (25) in seiner Breite verringert ist.

12. Einzelverpackung nach Anspruch 11, **dadurch gekennzeichnet, dass** der Halsbereich (8) eine obere Öffnung (9) ausbildet, die durch einen umlaufenden Rand (10) definiert ist und dass die Öffnung (9) elastisch aufweitbar ist.

13. Einzelverpackung nach Anspruch 11 oder 12, **dadurch gekennzeichnet, dass** die ovale Öffnung (9) des Halsbereichs (8) durch eine Schweißung oder Klebung oder Siegelung verschließbar ist.

14. Einzelverpackung nach einem der Ansprüche 1 bis 13, **dadurch gekennzeichnet, dass** der Verpackungskörper (1) in einem Stück im Hohlblasverfahren hergestellt ist und aus einem elastischen Kunststoff, bevorzugt einem TPU-Kunststoff, besteht.

15. Einzelverpackung nach einem der Ansprüche 1 bis 14, **dadurch gekennzeichnet, dass** sie aus einem Kunststoff mit einer Härte im Bereich von 45 bis 95 shore-A besteht und dabei besonders bevorzugt eine Härte von 85 shore-A +- 5 aufweist.

## Claims

1. An elastically expandable, sterilisable individual packaging for elongated objects (17) in the medical field, comprising a packaging body (1, 20) open at one end, which has a packaging chamber (27) closed at the bottom end and merging at its upper side into an elastically expandable closure section (6), **characterised in that** one or more locking studs (11, 12) are arranged in the transition region between the closure section (6) and the packaging chamber (27), which studs delimit the packaging chamber towards the top and form an elastically expandable closure gap (13, 13') between them, and **in that** a guide device (22), which is vertically aligned with the closure gap (13, 13'), is arranged within the packaging chamber (27) to guide the lower part of the packaged article (17) in a position-secured manner.

2. The individual packaging according to claim 1, **characterised in that** the packaging body (1, 20) is designed as an elastically expandable, flat, pouch-shaped plastic body.

3. The individual packaging according to claim 1 or 2, **characterised in that** the closure section (6) can be elastically expanded by finger pressure.

4. The individual packaging according to any one of claims 1 to 3, **characterised in that** at least two grip ribs (7) are arranged on the closure section (6) opposite one another on the narrow sides of the packaging body (1, 20), which are elastically deformable towards one another.

5. The individual packaging according to claim 4, **characterised in that** the locking studs (11, 12) for forming the closure gap (13) are arranged in the space between the opposing grip ribs (7).

6. The individual packaging according to any one of claims 1 to 5, **characterised in that** the packaging chamber (27) is designed to accommodate a single article (17) that is held by the guide device (22), and **in that** a free space (28) is present at an axial distance between the guide channel (23) and the locking studs (11, 12).

7. The individual packaging according to any one of claims 1 to 6, **characterised in that** at least one bottom-side stop stud (24) is arranged in the packaging chamber (27) in line with the guide channel (23) of the guide device (22), upon which the object (17) to be held rests with its lower end.

8. The individual packaging according to any one of claims 1 to 7, **characterised in that** the locking studs (11, 12) are each arranged individually or in pairs on the inner sides of the front wall (2) and the rear wall (4) and are aligned horizontally opposite one another, forming the elastically expandable closure gap (13).

9. The individual packaging according to any one of claims 1 to 8, **characterised in that** the guide device (22) consists of two parallel boundary ribs (23) spaced apart from one another, which together form a sleeve-like guide channel (23) for the longitudinal guidance of a part of the article (17) within the packaging chamber (27).

10. The individual packaging according to claim 9, **characterised in that** the boundary ribs (23) each connect the front wall (2) and the rear wall (4) of the packaging body (1) along their entire length.

11. The individual packaging according to any one of claims 1 to 10, **characterised in that** the transition from the oval packaging chamber (25) into the oval neck region (8) of the closure section (6) adjoining at the top is provided by an arcuate convex contour, such that the neck region (8) is narrower in cross-section than the packaging chamber (25).

12. The individual packaging according to claim 11, **characterised in that** the neck region (8) forms an upper opening (9) defined by a circumferential rim (10), and **in that** the opening (9) is elastically expandable.

13. The individual packaging according to claim 11 or 12, **characterised in that** the oval opening (9) of the neck region (8) can be closed by welding, gluing or sealing.

14. The individual packaging according to any one of claims 1 to 13, **characterised in that** the packaging body (1) is manufactured in one piece using a blow-moulding process and is made from an elastic plastic, preferably a TPU plastic.

15. The individual packaging according to any one of claims 1 to 14, **characterised in that** it is made from a plastic with a Shore A hardness in the range of 45 to 95 and particularly preferably with a Shore A hardness of 85 ± 5.

## Revendications

1. Emballage individuel stérilisable pouvant être élargi élastiquement pour des objets (17) allongés dans le domaine médical, constitué d'un corps d'emballage (1, 20) ouvert d'un côté, qui présente un espace d'emballage (27) fermé côté fond, qui passe sur sa face supérieure dans une pièce de fermeture (6) pouvant être élargie élastiquement, **caractérisé en ce que** dans la zone de transition entre la pièce de fermeture (6) et l'espace d'emballage (27) sont disposés un ou plusieurs boutons de blocage (11, 12), qui délimitent l'espace d'emballage vers le haut et qui forment entre eux une fente de fermeture (13, 13') pouvant être élargie élastiquement, et qu'un dispositif de guidage (22) aligné sur la fente de fermeture (13, 13') en ligne verticale pour le guidage en position sécurisée de la partie inférieure de l'objet (17) emballé est disposé dans l'espace d'emballage (27).

2. Emballage individuel selon la revendication 1, **caractérisé en ce que** le corps d'emballage (1, 20) est réalisé en tant que corps plastique plat en forme de poche pouvant être élargi élastiquement.

3. Emballage individuel selon la revendication 1 ou 2, **caractérisé en ce que** la pièce de fermeture (6) peut être élargie élastiquement à la force du doigt.

4. Emballage individuel selon l'une des revendications 1 à 3, **caractérisé en ce que** sur la pièce de fermeture (6) sont disposées au moins deux nervures de préhension (7), disposées en vis-à-vis sur les côtés étroits du corps d'emballage (1, 20), qui sont déformables élastiquement l'une en direction de l'autre.

5. Emballage individuel selon la revendication 4, **caractérisé en ce que** les boutons de blocage (11, 12) pour la réalisation de la fente de fermeture (13) sont disposés dans l'espace intermédiaire entre les nervures de préhension (7) opposées l'une à l'autre.

6. Emballage individuel selon l'une des revendications 1 à 5, **caractérisé en ce que** l'espace d'emballage (27) est réalisé pour recevoir un seul objet (17) qui est maintenu par le dispositif de guidage (22) et qu'un espace libre (28) est présent dans l'écart axial entre le canal de guidage (23) et les boutons de blocage (11, 12).

7. Emballage individuel selon l'une des revendications 1 à 6, **caractérisé en ce que** dans l'espace d'emballage (27) est disposé, dans un prolongement aligné du canal de guidage (23) du dispositif de guidage (22), au moins un picot de butée (24) côté fond, sur lequel repose l'objet (17) à maintenir avec son extrémité inférieure.

8. Emballage individuel selon l'une des revendications 1 à 7, **caractérisé en ce que** les boutons de blocage (11, 12) sont disposés chacun, individuellement ou par paires, sur les faces intérieures de la paroi avant (2) et de la paroi arrière (4) et sont alignés en regard l'un par rapport à l'autre dans la direction horizontale et forment la fente de fermeture (13) pouvant être élargie élastiquement.

9. Emballage individuel selon l'une des revendications 1 à 8, **caractérisé en ce que** le dispositif de guidage (22) est constitué de deux nervures de limitation (23) parallèles l'une à l'autre et réalisant un écart réciproque l'une par rapport à l'autre, qui forment entre elles un canal de guidage (23) en forme de douille pour le guidage longitudinal d'une partie de l'objet (17) dans l'espace d'emballage (27).

10. Emballage individuel selon la revendication 9, **caractérisé en ce que** les nervures de limitation (23) relient respectivement la paroi avant (2) et la paroi arrière (4) du corps d'emballage (1) sur toute leur longueur.

11. Emballage individuel selon l'une des revendications 1 à 10, **caractérisé en ce que** la transition de l'espace d'emballage (25) ovale dans la zone de col (8) ovale de la pièce de fermeture (6) se raccordant par le haut s'effectue par un contour convexe en forme d'arc, de telle sorte que la zone de col (8) soit réduite en largeur dans la section transversale par rapport à l'espace d'emballage (25).

12. Emballage individuel selon la revendication 11, **caractérisé en ce que** la zone de col (8) réalise une ouverture (9) supérieure qui est définie par un bord périphérique (10) et que l'ouverture (9) peut être élargie élastiquement.

13. Emballage individuel selon la revendication 11 ou 12, **caractérisé en ce que** l'ouverture (9) ovale de la zone de col (8) peut être fermée par une soudure ou un collage ou un scellement.

14. Emballage individuel selon l'une des revendications 1 à 13, **caractérisé en ce que** le corps d'emballage (1) est fabriqué en une seule pièce par procédé de soufflage creux et est constitué d'un plastique élastique, de préférence un plastique TPU.

15. Emballage individuel selon l'une des revendications 1 à 14, **caractérisé en ce qu'**il est constitué d'un plastique d'une dureté dans la plage de 45 à 95 shore-A et présente de manière particulièrement préférée une dureté de 85 shore-A +- 5.
